# EUROPEAN PATENT SPECIFICATION

(11) **EP 0 831 850 B1**
(45) Date of publication and mention of the grant of the patent: **03.03.2004**
(21) Application number: 96919031.3
(22) Date of filing: 03.06.1996
(51) Int. Cl.: A61K 31/70, A61P 17/02, A61P 17/06, A61P 17/16

(54) **USE OF LOCALLY APPLIED DNA FRAGMENTS**
VERWENDUNG VON LOKALEN APPLIZIERTEN DNA-FRAGMENTEN
UTILISATION DE FRAGMENTS D'ADN PAR APPLICATION LOCALE

(30) Priority: 06.06.1995 US 467012
(43) Date of publication of application: 01.04.1998
(73) Proprietor: TRUSTEES OF BOSTON UNIVERSITY, Boston, MA 02115 (US)
(72) Inventor: GILCHREST, Barbara, A., Brookline, MA 02146 (US); ELLER, Mark, Boston, MA 02116 (US); YAAR, Mina, Sharon, MA 02067 (US)
(74) Representative: Price, Vincent Andrew
(86) International application number: PCT/US1996/008386
(87) International publication number: WO 1996/039152

(56) References cited:
- WO-A-95/01773
- ANNUAL MEETING OF THE SOCIETY FOR INVESTIGATIVE DERMATOLOGY, WASHINGTON, D.C., USA, MAY 1-5, 1996. JOURNAL OF INVESTIGATIVE DERMATOLOGY 106 (4). 1996. 808, XP002014517 MAGNONI C ET AL: "Thymidine dinucleotides enhance DNA repair in normal human skin cells."
- ANNUAL MEETING OF THE SOCIETY FOR INVESTIGATIVE DERMATOLOGY, WASHINGTON, D.C., USA, MAY 1-5, 1996. JOURNAL OF INVESTIGATIVE DERMATOLOGY 106 (4). 1996. 835, XP002014518 ELLER M S ET AL: "Activation of p53 tumor supressor protein by thymidine dinucleotides."

## Description

### Background of the Invention

Human skin consists of two layers, the dermis and the epidermis. The epidermis, which is the uppermost of the two skin layers, encompasses many different cell types, including melanocytes and keratinocytes. Melanocytes are specialized cells in the basal layer of the epidermis which synthesize melanin; the melanin is then packaged into melanosomes and then transported into keratinocytes.

Exposure of skin to the sun results in vitamin D synthesis, sunburn (erythema), and tanning, the skin's major form of endogenous protection against subsequent skin damage from ultraviolet (UV) irradiation. Various morphologic and enzymatic changes occur at the cellular level in epidermal melanocytes in response to UV irradiation. Melanin, which is increased in "tanned" skin, serves as a filter with absorbance within the UV range and provides photoprotection for the individual.

The peak action spectrum for erythema is in the UV-B range, 290-305 nm. UV-B rays are absorbed by proteins and nucleic acids of the epidermis, causing the production of thymine dimers, which are known to be formed by UV irradiation of nuclear DNA and to be excised from the DNA strand by the action of highly specific enzymes, including endonucleases. If not removed, these dimers can stall DNA replication forks generating regions of single-stranded DNA. Failure to remove thymine dimers and other DNA mutations in the genome may lead to somatic mutations resulting in carcinogenesis.

In bacteria it is known that the DNA fragments released from stalled replication forks can interact with nuclear proteins which then regulate the expression of specific genes in the DNA as part of the organism's SOS response to UV damage. The tanning response of skin might reasonably be considered part of the analogous SOS response in mammalian skin. The precise stimulus for UV-induced tanning, however, remains unknown.

UV irradiation is successfully used in phototherapy and photochemotherapy for certain dermatological conditions. For example, psoriasis is a common dermatologic disease affecting 1 to 2 percent of the population. Psoriasis can be treated with UV-B irradiation, either alone or in conjunction with agents such as coal tar or anthralin, or with UV-A irradiation in combination with psora lens (PUVA therapy). Other diseases which respond to UV irradiation treatment include atopic dermatitis and vitiligo. Despite the benefits of phototherapy and photochemotherapy, these treatments carry the same risks as chronic exposure to sun, including wrinkling, "photoaging," and skin cancer.

### Summary of the Invention

The current invention pertains to the use of DNA fragments for the manufacture of a medicament for treating or preventing hyperproliferative diseases or pre-cancerous conditions affecting epithelial cells, such as psoriasis or other skin diseases, including hyperproliferative, pre- cancerous or UV-induced dermatoses, in a mammal.

The DNA fragments are either single- or double-stranded, or a mixture of both single- and double-stranded DNA fragments, or deoxynucleotides, dinucleotides, or dinucleotide dimers, such that the DNA fragments, deoxynucleotides, dinucleotides, or dinucleotide dimers. The DNA fragments can be delivered to epithelial cells of a mammal either topically, orally, by aerosol, or by any other appropriate means, such as by instillation. The DNA fragments, either single- or double- stranded, or a mixture of both single- and double-stranded DNA fragments, or deoxynucleotides or dinucleotides can be ultraviolet-irradiated.

### Brief Description of the Figures

Figure 1 is a graphic representation of the cell growth rate of human squamous carcinoma cells dosed with water (diluent), 100 µM pTpT (T₂) or 100 µM pdApdA (A₂). Day 0 is before dosage; days 1, 3, 4 and 5 are days after dosage.
Figure 2 is a graphic representation of the cell growth rate of normal human fibroblasts dosed with water (diluent) or 100 µM pTpT (T₂) .Day 0 is before dosage; days 1, 3, 4 and 5 are days after dosage. Values represent averages ± standard deviations of duplicate cultures.
Figure 3 is a graphic representation of the cell growth rate of human cervical carcinoma cells dosed with either water (diluent) or 100 µM pTpT (T₂). Day 0 is before dosage; days 1, 4 and 6 are days after dosage.
Figure 4 is a graphic representation of the cell yield of human melanoma cell lines dosed with either diluent or 100 µM pTpT (T₂).
Figure 5 is a graphic representation of the cell growth rate of normal human keratinocytes dosed with water (diluent) or 100 µM pTpT (T₂). Day 0 is before dosage; 8, 24, 48 and 7 2 are hours after dosage. Values represent averages ± standard deviations of duplicate cultures.
Figure 6 is a graphic representation of the average cell number of human neonatal fibroblasts dosed with either water, T₂ or A₂.
Figure 7 is a graphic representation of the average cell number of human neonatal fibroblasts dosed with either water, T₂ or A₂.
Figure 8 is a graphic representation of the cell growth rate of normal human fibroblasts dosed with water (diluent) or 100 µM pTpT (T₂). Day 0 is before dosage; 8, 24, 48 and 72 are hours after dosage. Values represent averages ± standard deviations of duplicate cultures.
Figure 9 is a graphic representation of the cell growth rate of p53-null H1299 lung carcinoma cells dosed with water (diluent) or 100 µM pTpT (T₂). Day 0 is before dosage; 1, 2, 3 and 4 are days after dosage. Values represent averages ± standard deviations of duplicate cultures.
Figure 10 is a graphic representation of enhancement of DNA repair of a reporter plasmid in human keratinocytes treated with pTpT. Open boxes, sham-irradiated control plasmid; filled boxes, UV-irradiated plasmid.
Figure 11 is a graphic representation of enhancement of DNA repair of a reporter plasmid in human fibroblasts treated with pTpT. Open boxes, sham-irradiated control plasmid; filled boxes, UV-irradiated plasmid.

### Detailed Description of the Invention

The invention pertains to use of DNA fragments for the manufacture of a medicament for the prevention or treatment of certain hyperproliferative diseases or pre- cancerous conditions affecting epithelial cells, including skin diseases such as psoriasis and hyperproliferative, pre-cancerous or UV-induced dermatoses, in mammals, and particularly in humans. The invention further pertains to use of DNA fragments, deoxynucleotides, dinucleotides, or dinucleotide dimers, for reduction of photoaging or prophylaxis against or reduction in the likelihood of the development of skin cancer, in a mammal.

In one embodiment of the invention, DNA fragments of approximately 3-200 bases in length, deoxynucleotides (single bases), dinucleotides, or dinucleotide dimers, are administered to the mammal in an appropriate vehicle. As used herein, "DNA fragments" refers to single-stranded DNA fragments, double-stranded DNA fragments, or a mixture of both single- and double-stranded DNA fragments. "Deoxynucleotides" refers to either a single type of deoxynucleotide or a mixture of different deoxynucleotides.

"Dinucleotides" can comprise a single type of nucleotide or different types of nucleotides, and can comprise a mixture of different types of dinucleotides. In a preferred embodiment, the nucleotides of the dinucleotides are deoxynucleotides. Representative dinucleotides include d(pT)₂, d(pC)₂, d(pA)₂, d(pCpT), d(pTpC), d(CpT) , d(TpC) and d(TpT), where T is thymine, C is cytosine, d is deoxy, and p is phosphate (see Niggli, *Pbotocbem. Pbotobiol*. *38(3)*:353-356 (1988)). A combination of at least two or more of DNA fragments, deoxynucleotides, dinucleotides, and/or dinucleotide dimers can also be used. The DNA fragments, deoxynucleotides, or dinucleotides can be ultraviolet-irradiated. Such ultraviolet irradiation results in photodimerization between two adjacent pyrimidine residues (i.e., thymine (T) and cytosine (C)) present in the DNA fragments or dinucleotides.

The DNA fragments, deoxynucleotides, dinucleotides, or dinucleotide dimers can be obtained from any appropriate source, or can be synthetic DNA fragments, deoxynucleotides, dinucleotides, or dinucleotide dimers. For example, salmon sperm DNA can be dissolved in water, and then the mixture can be autoclaved to fragment the DNA.

The DNA fragments, deoxynucleotides, dinucleotides or dinucleotide dimers, can be applied alone or in combination with other compounds, such as perfumes or colorants. They can be applied in a vehicle, such as water, saline, or in another appropriate delivery vehicle. The delivery vehicle can be any appropriate vehicle which delivers the DNA fragments, deoxynucleotides, dinucleotides, or dinucleotide dimers. In one embodiment, propylene glycol is used as a delivery vehicle. In a preferred embodiment, a mixture of propylene glycol:ethanol:isopropyl myristate (1:2.7:1) containing 3% benzyl sulfonic acid and 5% oleyl alcohol is used. In another embodiment, a liposome preparation is used. The liposome preparation can be comprised of any liposomes which penetrate the stratum corneum and fuse with the cell membrane, resulting in delivery of the contents of the liposome into the cell. For example, liposomes such as those described in U.S. Patent No. 5,077,211 of Yarosh, U.S. Patent No. 4,621,023 of Redziniak et al. or U.S. Patent No. 4,508,703 of Redziniak et al. can be used.

The delivery vehicle can contain perfumes, colorants, stabilizers, sunscreens, or other ingredients.

The DNA fragments, deoxynucleotides, dinucleotides, or dinucleotide dimers, are applied (administered) to the epithelial cells of interest in an appropriate manner. The "cells of interest", as used herein, are those cells which may become affected or are affected by the hyperproliferative disease or precancerous condition. In one embodiment, the DNA fragments, deoxynucleotides, dinucleotides, or dinucleotide dimers are applied topically to the skin surface. In other embodiments, the DNA fragments, deoxynucleotides, dinucleotides, or dinucleotide dimers, are delivered orally to the oral or intestinal epithelium; by aerosol to the respiratory epithelium; by instillation to the bladder epithelium; or by other means to other cells or tissues in the body. The DNA fragments, deoxynucleotides, dinucleotides, or dinucleotide dimers, are applied at an appropriate time, in an effective amount. The "appropriate time" will vary, depending on the type and molecular weight of the DNA fragments, deoxynucleotides, dinucleotides, or dinucleotide dimers, or agent, employed; the condition to be treated or prevented; the results sought; and the individual patient. An "effective amount", as used herein, is a quantity or concentration sufficient to achieve the desired result. The effective amount will depend on the type and molecular weight of the DNA fragments, deoxynucleotides, dinucleotides, or dinucleotide dimers, or agent, employed; the condition to be treated or prevented; the results sought; and the individual patient. For example, for the treatment or prevention of psoriasis, or for hyperproliferative, pre-cancerous, or UV-induced dermatoses, the effective amount is the amount necessary to relieve the symptoms of the disease, to reduce the area of skin affected by the disease, or to prevent the formation of affected areas. The concentration will generally be approximately 2-300 µm, and will depend on the type and molecular weight of the DNA fragments, deoxynucleotides, dinucleotides, or dinucleotide dimers, or agent, employed; the condition to be treated or prevented; the results sought; and the individual patient. In a preferred embodiment, the concentration is 50-200 µm; in a more preferred embodiment, the concentration is 75-150 µm. In a first embodiment of the current invention, DNA fragments, such as single-stranded DNA fragments, double- stranded DNA fragments, a mixture of single- and double- stranded DNA fragments, deoxynucleotides, dinucleotides, or dinucleotide dimers, are applied, either without a vehicle or in an appropriate delivery vehicle, to the epithelial cells of interest in the mammal in order to treat or prevent a hyperproliferative disease affecting epithelial cells. The DNA fragments, deoxynucleotides, dinucleotides, or dinucleotide dimers, can be applied solely to affected areas, or can be applied prophylactically to regions commonly affected by the hyperproliferative disease.

In a preferred embodiment of the invention, the DNA fragments, deoxynucleotides, dinucleotides, or dinucleotide dimers, are applied, either without a vehicle or in an appropriate delivery vehicle, to the epidermis for the treatment or prevention of psoriasis. The DNA fragments, deoxynucleotides, dinucleotides, or dinucleotide dimers, can be applied solely to affected areas, or can be applied prophylactically to regions of epidermis commonly affected.

In another preferred embodiment of the invention, the DNA fragments, deoxynucleotides, dinucleotides, or dinucleotide dimers, are applied, either without a vehicle or in an appropriate delivery vehicle, to the epidermis for the treatment or prevention of atopic dermatitis. The DNA fragments, deoxynucleotides, dinucleotides, or dinucleotide dimers, can be applied solely to affected areas, or can be applied prophylactically to regions of epidermis commonly affected. In another preferred embodiment of the invention, the DNA fragments, deoxynucleotides, dinucleotides, or dinucleotide dimers, are applied, either alone or in an appropriate delivery vehicle, to the epidermis for the treatment or prevention of vitiligo. The DNA fragments, deoxynucleotides, dinucleotides, or dinucleotide dimers, can be applied solely to affected areas, or can be applied prophylactically to regions of epidermis commonly affected.

In another preferred embodiment, DNA fragments, deoxynucleotides, dinucleotides, or dinucleotide dimmers, are applied, either alone or in an appropriate delivery vehicle, to the epidermis for the treatment or prevention of other hyperproliferative, pre-cancerous or UV-induced dermatoses.

In a second embodiment, DNA fragments, deoxynucleotides, dinucleotides, or dinucleotide dimers, are applied, either alone or in an appropriate delivery vehicle, to the epidermis for prophylaxis against or reduction in the likelihood of development of skin cancer. The DNA fragments, deoxynucleotides, dinucleotides, or dinucleotide dimers, are applied at an appropriate time (i.e., sufficiently close in time to exposure of the skin to UV irradiation) : the DNA fragments, deoxynucleotides, dinucleotides, or dinucleotide dimers can be applied before, during or after exposure to UV irradiation. They can be applied daily or at regular or intermittent intervals. In a preferred embodiment, the DNA fragments, deoxynucleotides, dinucleotides, or dinucleotide dimers, can be applied on a daily basis to skin which may be exposed to sunlight during the course of the day.

The invention is further illustrated by the following Examples.

### EXAMPLE 1 Application to Human Squamous Carcinoma Cells

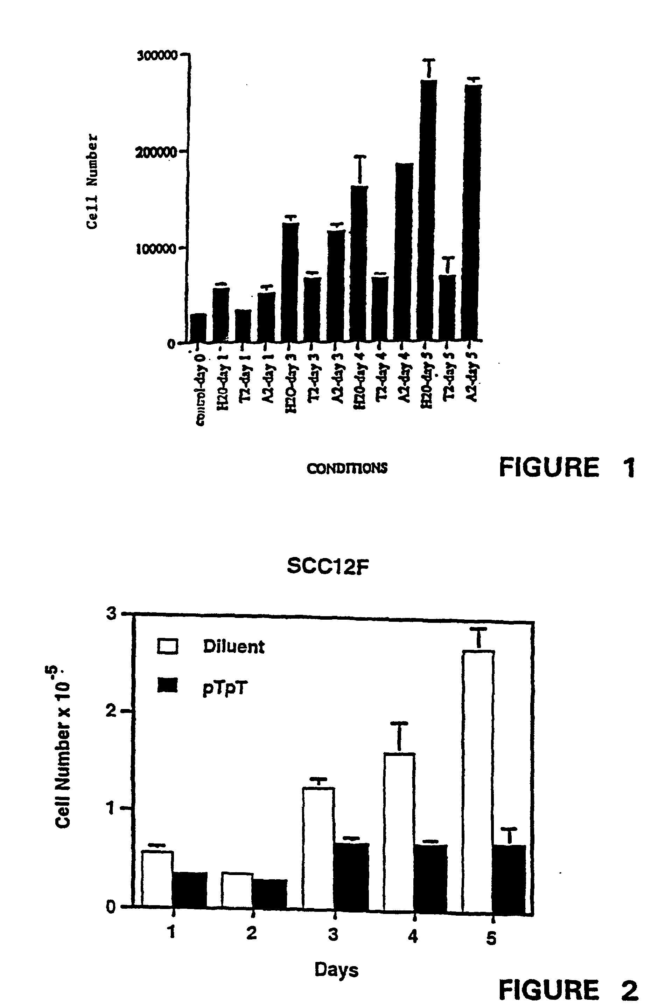

Human squamous carcinoma cells line SCC12F cells were maintained in primary keratinocyte medium (300 ml DME, 100 ml F-12 nutrient supplement, 50 ml 10x Adenine, 50 ml fetal bovine serum, 5 ml penicillin/streptomycin stock, and 0.5 ml of 10 µg/ml epidermal growth factor and hydrocortisone to final concentration of 1.4 µg/ml) and dosed with either water (diluent), 100 µM pTpT (T₂, Midland Certified Reagent Company, Midland, TX) or 100 µM pdApdA (A₂). Cells were harvested before dosing (day 0), and 1, 3, 4, and 5 days after dosage, and were counted by Coulter counter. After harvesting, the cells were processed for total RNA isolation and were analyzed by Northern blot. Addition of pTpT (T₂) to human squamous carcinoma cells resulted in marked decreases in cell growth rate, as shown in Figure 1. Addition of a control deoxyadenine dinucleotide (pdApdA or A₂), a compound very similar to pTpT but not readily dimerized by UV irradiation and therefore not excised during the course of UV-induced DNA repair, has no effect (A).

In a second experiment, SCC12F cells were cultured as described above. Two or three days after seeding, the preconfluent cultures were given fresh medium supplemented with either 100 µM T₂ or diluent as a control. Cells were collected by trypsinization daily and counted by Coulter counter. The cell yield in cultures treated with T₂ was reduced by 75% compared to that of paired control cultures after five days (Figure 2). This corresponds to 2.3 population doublings in this time for control cells, compared with 1 doubling for T₂-treated cells. These results further demonstrate that application of the DNA fragments inhibits cell multiplication.

In a third experiment, it was demonstrated that addition of thymidine dinucleotides (T₂) to human squamous carcinoma cells for 24-72 hours resulted in upregulation of at least three genes: growth arrest and DNA damage (GADD 45), senescence-derived inhibiter (Sdi I), and excision repair cross-complementing (ERCC-3) (data not shown). Paired cultures of SCC12F cells were maintained in a Dulbecco's modified Eagle's Medium (DMEM; GIBCO/BRL, Gaithersburg, MD)-based keratinocyte growth medium supplemented with 10% fetal calf serum (Hyclone Labs, Logan, UT) and epidermal growth factor as described (Hollander, M.C. et al., J. Biol. Chem. 268:328-336 (1992)). Pre-confluent cultures were given fresh medium supplemented with either 100 µM pTpT, or an equal volume of diluent. Cells were collected daily after additions and processed for total RNA isolation using the Tri-Reagent extraction method (Molecular Research center, Cincinnati, OH) following the protocol of the manufacturer. Ten micrograms of RNA from each sample was gel electrophoresed, transferred to a nylon filter and probed as described previously (Nada, A. et al., Exp. Cell Res. 211:90-98 (1994)). The cDNA for GADD 45 was generated by PCR using primers based on the human GADD 45 gene sequence (Mitsudomi, T. et al., Oncogene 7:171-180 (1992)). The cDNA for ERCC 3 was purchased from the American Type Culture Collection (ATCC, Rockville, MD). The SDI 1 cDNA was a gift of Dr. J. Smith and has been described previously (Walworth, N.C. and Bernards, R., Science 271:353-356 (1996)).

Compared to the diluent control, the mRNAs for GADD 45, ERCC 3 and SDI 1 were up-regulated in pTpT-treated cells as early as 24 hours, and remained elevated for several days. Addition of the control deoxyadenine dinucleotide (A₂) was less effective or ineffective in inducing these genes (data not shown). Comparable data have been obtained in preliminary experiments with S91 melanoma cells, and normal human fibroblasts (data not shown).

The time course of induction is similar to that observed after UV irradiation for the two genes for which this has been studied (GADD 45 and Sdi I) (Fornace, A.J. et al., Proc. Natl. Acad. Sci. USA 85:8800-8804 (1988); Hollander, M.C. et al., J. Biol. Chem. 268:24385-24393 (1993); Zhan, Q. et al., Mol. Cell Biol. 14:2361-2371 (1994); El-Deiry, W.S. et al., Cancer Res. 54:1169-1174 (1994); and El-Deiry, W.S. et al., Cell 75:817-825 (1993)) and also similar to the time course of induction of the tyrosinase gene by T₂ in melanocytes and melanoma cells (Maltzman, W. and L.Czyzyk, Mol. Cell Biol. 4:1689-1694 (1984); and Lu, X. and D.P. Lan, Cell 75:765-778 (1993)). Sdi I is known to be involved in cell cycle regulation and specifically in blocking cell division. GADD 45 and ERCC-3, a human DNA repair enzyme, are known to be involved in repair of UV-induced DNA damage. The response to pTpT is identical to that observed after UV irradiation of these cell lines, and is also similar to the response to various antimetabolites, such as methotrexate, that are clinically effective in the treatment of hyperproliferative skin disorders.

### EXAMPLE 2 Application to Human Cervical Carcinoma Cells

Human cervical carcinoma cells (HeLa cells) were maintained in DME + 10% calf serum and dosed with either water (diluent) or 100 µM pTpT (T2). Cells were collected 1, 4 and 6 days after dosage and counted by Coulter counter.

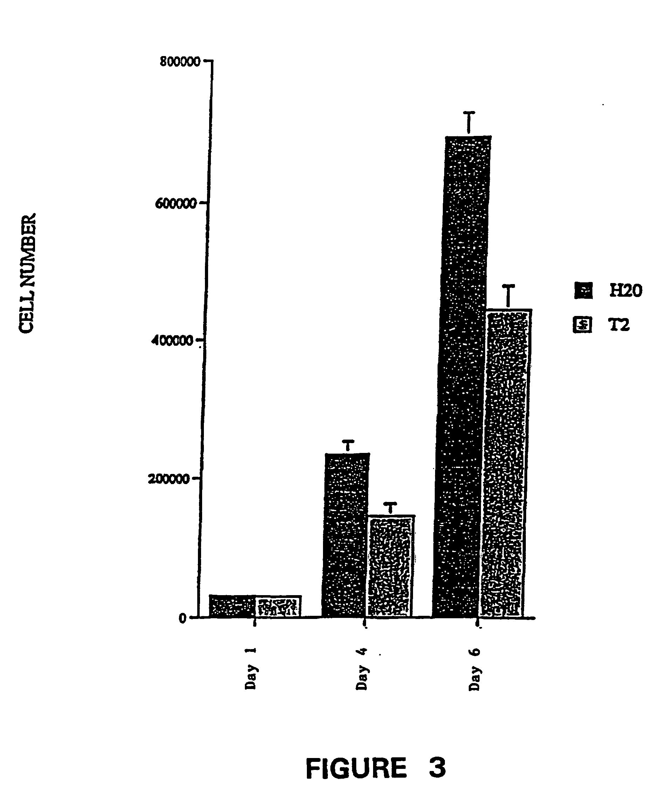

Addition of pTpT (T₂) to the human cervical carcinoma cells resulted in marked decreases in cell growth rate, as shown in Figure 3.

### EXAMPLE 3 Application to Human Melanoma Cells

Human melanoma cell lines CRL 1424, Malma, Sk Mel 2, and Sk Mel 28 were obtained from the American Type Culture Collection (ATCC). The cell lines were maintained in DME + 2% calf serum, and dosed with either water (diluent) with DME, or 100 µM pTpT (T2) in DME. One week after dosage, cells were collected and counted by Coulter counter.

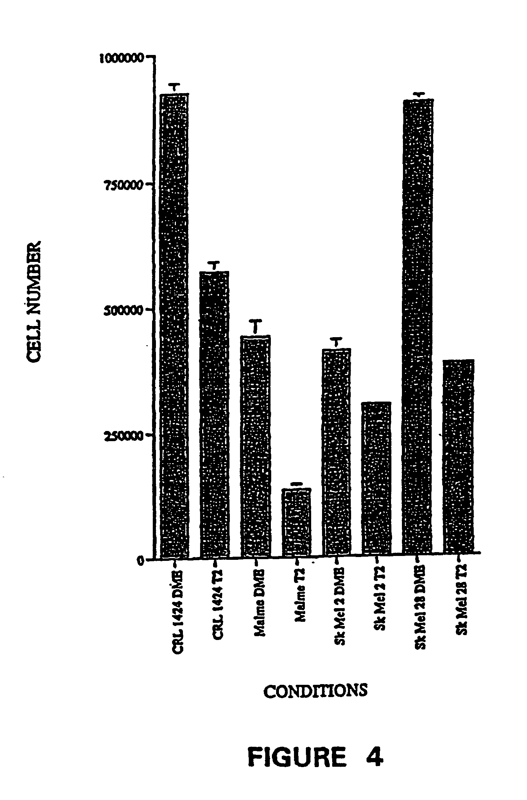

Addition of pTpT (T₂) to any of the four different human melanoma cell lines results in marked decreases in cell yields, as shown in Figure 4.

### EXAMPLE 4 Application to Human Keratinocytes

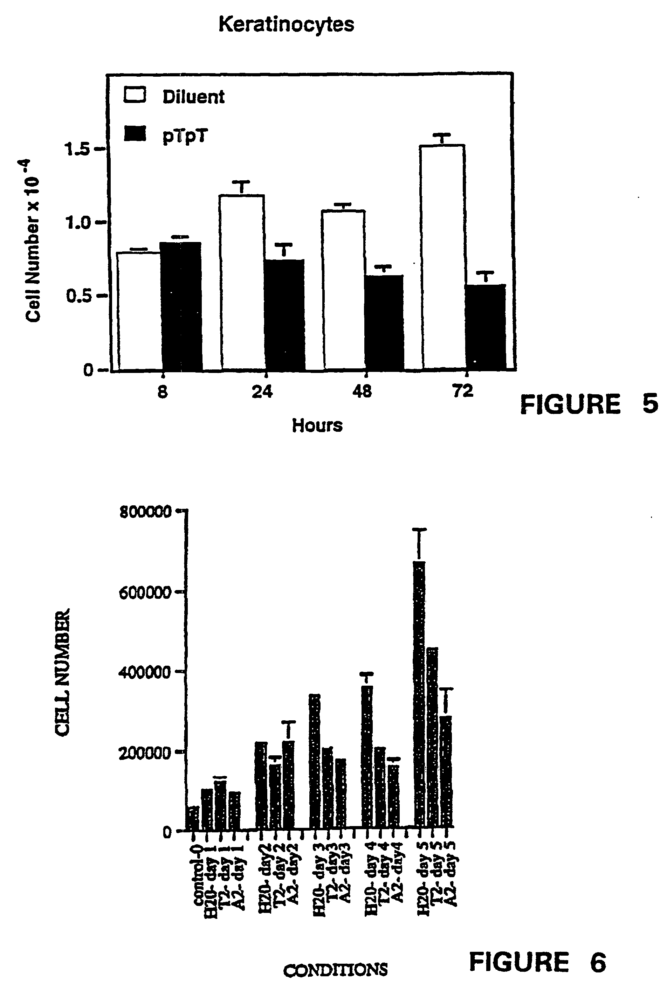

Normal human neonatal keratinocyte cells were cultured as described above in Example 1 for SCC12F cells, and treated with either 100 µM T₂ or diluent as a control. Cells were harvested for cell counts. The cell yield in cultures treated with T₂ was reduced by 63% compared to that of paired control cultures after three days (Figure 5). This corresponds to one population doubling in this time for control cells, while the number of T₂-treated cells remained the same. These results demonstrate that application of the DNA fragments inhibits cell multiplication.

Northern blot analysis of the normal human keratinocytes treated with pTpT for 24-72 hours that shows induction of the tumor necrosis factor (TNF) alpha gene (data not shown). This immunomodulatory cytokine, known to be induced by UV irradiation, may thus be induced by pTpT. Use of locally applied DNA fragments, deoxynucleotides, dinucleotides, or dinucleotide dimers may therefore be useful in immunodulation of cutaneous reactions and in treatment or prevention of diseases or conditions involving immunomodulation.

### EXAMPLE 5 Inhibition of Cell Growth of Normal Neonatal Fibroblasts by DNA Fragments

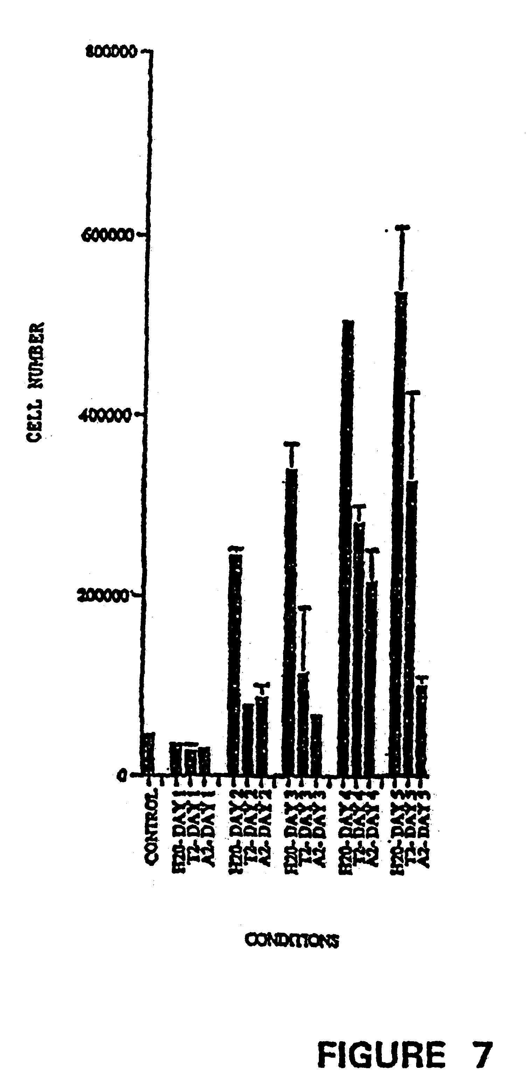

Normal human neonatal fibroblasts were plated in Falcon P35 culture dishes at a density of 9 x 10⁴ cells/dish. The culture medium was DME + 10% calf serum, 2 ml per plate. One day after plating, cultures were supplemented with either 100 µl 2 mM T₂ in DME or 100 µl 2 mM A₂ in DME, or water (control). Two plates were collected and counted before the additions to give a starting, or "day 0," reading. Duplicate plates of each condition were harvested through five days after addition of the supplements and cell number determined. All cell counts were done by Coulter Counter. Results are shown in Figures 6 and 7. The results indicate that application of the DNA fragments inhibits cell multiplication.

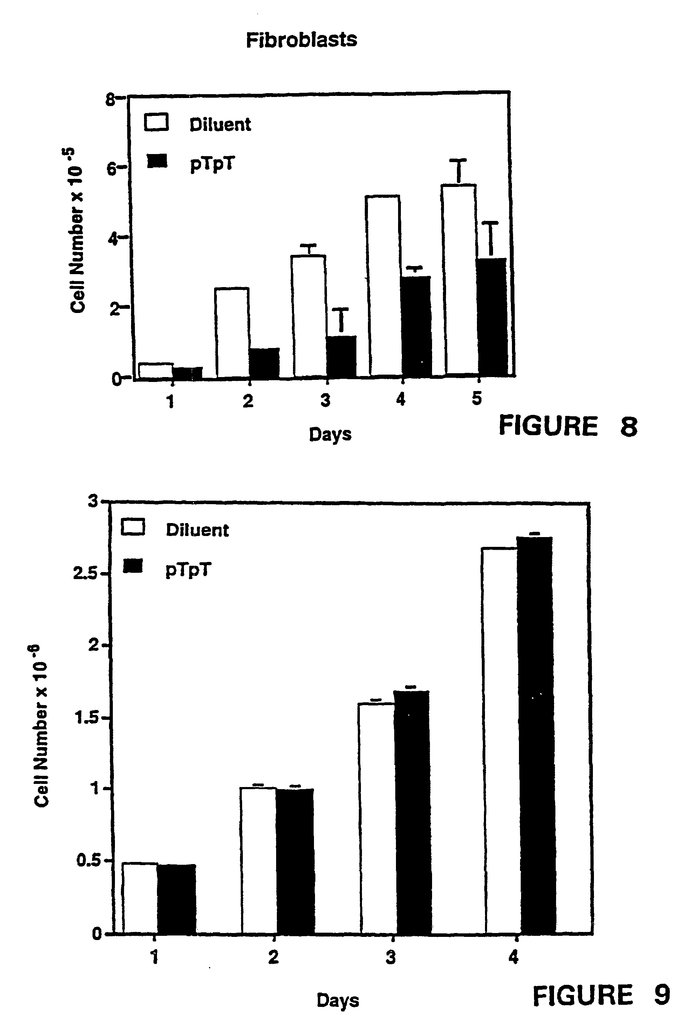

In a second experiment, normal human neonatal fibroblasts were plated and cultured, as described above in Example 1 for SCC12F cells. Cultures were supplemented with either 100 µl 2 µM T₂ or water (control), and cells were harvested for cell counts. The cell yield in fibroblast cultures treated with T₂ was reduced by 40% compared to that of paired control cultures after three days (Figure 8). This corresponds to 4 population doublings in this time for control cells, compared with 3.6 doublings for T₂-treated cells. These results further demonstrate that application of the DNA fragments inhibits cell multiplication.

### EXAMPLE 6 Effect of pTpT Applications on Epidermal Labeling Index

Guinea pigs received one or two daily topical applications of 100 µM pTpT, or vehicle alone as control, for three days. On the fourth day, punch biopsies were obtained and maintained for 7 or 8 hours in primary keratinocyte medium supplemented with 10 uCi/ml ³H-thymidine (specific activity 9.0 ci/m mole, NEN). Tissues were then rinsed with cold medium and fixed in 10% phosphate buffered formalin. After a series of dehydration steps, tissues were embedded in paraffin. 6 um sections were cut and mounted onto glass slides, dipped in NTB-2 Nuclear Track emulsion and kept in the dark at 4°C for 7 days. Sections were developed in Kodak D-19 developer and stained with hematoxylin and eosin. Labeling index was measured by calculating the percentage of labeled nuclei among 100 basal keratinocytes.

### Results:

| | **Labeling Index** |
|---|---|
| | **2 daily applications** |
| Vehicle control | pTpT |
| 4 ± 1.4 | 1.5 ± 0.7 |
| | |

| | **1 daily application** |
|---|---|
| Vehicle control | pTpT |
| 4.5 ± 2.1 | 2 ± 0 |

Results ± SD are shown.

Labeling index (a measure of epidermal turnover rate) is less in pTpT-treated skin than in vehicle-treated skin, (>0.03 paired T test) in both experiments. These results demonstrate that the DNA fragments reduce epidermal turnover rate.

### EXAMPLE 7 Role of p53 in DNA Repair

Both the GADD 45 and SDi 1 genes are known to be transcriptionally regulated by the tumor suppressor protein p53 (Kastan, M.B. et al., Cell 71:587-597 (1992); El-Deiry, W.S. et al., Cell 75:817-825 (1993)). After UV- and γ-irradiation, as well as treatment of cells with DNA-damaging chemical agents, there is a rapid stabilization and nuclear accumulation of p53 (Fritsche, M. et al., Oncogene 8:307-318 (1993); Nelson, W.G. and Kastan, M.B., Mol. Cell. Biol. 14:1815-1823 (1994); Lu, X. and Lane, D.P., Cell 75:765-778 (1993)), after which this protein binds to specific promoter consensus sequences and modulates the transcription of regulated genes (Lu, X. and Lane, D.P., Cell 75:765-778 (1993)). Recent data suggest that p53 can also be activated by the binding of small single-stranded DNAs, as well as certain peptides and antibodies, to a carboxyl terminal domain of this protein (Jayaraman, L. and Prives, C., Cell 81:1021-1029 (1995); Hupp, T.R. et al., Cell 83:237-245 (1995)). In order to determine whether the inhibitory effect of the dinucleotide pTpT on cell proliferation is mediated through p53, the growth response of a p53 null cell line, H1299 lung carcinoma cells, was examined. The p53-null H1299 cells (Sanchez, Y. et al., Science 271:357-360 (1996)) was maintained in DMEM with 10% calf serum. Preconfluent cultures were given fresh medium supplemented with either 100 µM pTpT or diluent. Cells were collected on consecutive days by trypsinization, and counted by Coulter counter. As shown in Figure 9, there was no inhibition of proliferation of pTpT-treated H1299 cells compared to diluent-treated controls.

The effect of pTpT on the level and intracellular distribution of p53 in normal neonatal fibroblasts was examined by immunoperoxidase staining using a p53-specific monoclonal antibody (mAb 421, Oncogene, Cambridge, MA).

Preconfluent cultures were treated with either 100 µM pTpT or diluent for 24 hours before cell staining. Cells were first fixed for one minute in Histochoice fixative (Amresco, Solon, OH) followed by a five-minute rinse in PBS. p53 was detected using the Vectastain Elite ABC kit (Vector Laboratories, Burlingame, CA) and the p53-specific monoclonal antibody mAb 421. Within 24 hours, an increase in intranuclear p53 was detected in pTpT-treated cells compared to diluent-treated cells (data not shown), as has been reported after UV-irradiation (Fritsche, M. et al., Oncogene 8:307-318 (1993); Nelson, W.G. and Kastan, M.B., Mol. Cell. Biol. 14:1815-1823 (1994); Lu, X. and Lane, D.P., Cell 75:765-778 (1993)). These results are consistent with the induction of the p53-regulated genes GADD 34 and SDI 1 in fibroblasts (data not shown) as well as in SCC12F cells, by pTpT.

In another experiment, pTpT was found to induce the expression of SDI 1 mRNA in a p53-dependent manner. Preconfluent cultures of H1299 cells were transfected with an expression vector containing the wild type human p53 cDNA under the control of the human cytomegalovirus promoter/enhancer (Dr. Bert Vogelstein, Johns Hopkins Oncology Center). Control transfections were performed using the vector from which the p53 cDNA was removed. Transfections were carried out using the Lipofectin Reagent Kit (GIBCO/BRL). One day after transfection, cells were collected for Western blot analysis using 20 µg total protein as described (Yaar, M. et al., J. Clin. Invest. 94:1550-1562 (1994)). p53 was detected using mAb 421, anti-mouse Ig linked to horseradish peroxidase (Amersham, Arlington Heights, IL) and an ECL-kit (Amersham) following the directions of the manufacturer. At the time of protein collection, duplicate cultures of H1299 cells transfected with the p53 expression vector (designated "p53") or control vector ("Ctrl") were given either diluent (DMEM) or 100 µM pTpT. After 24 hours, the cells were collected, processed for RNA isolation and Northern blot analysis with an SDI 1 cDNA probe. The autoradiograph was scanned using a Macintosh IIsi computer and Macintosh One scanner, and the brightness and contrast were adjusted to display differences in autoradiographic signals maximally. The results indicated that p53-null H1299 cells express a very low level of the SDI 1 transcript and this level is not affected by addition of pTpT (data not shown).
Transfection of these cells with a wild-type p53 expression vector increased the level of SDI 1 and rendered this transcript inducible by addition of pTpT (data not shown). Western analysis confirmed that H1299 cells normally express no p53 and that transfected H1299 cells expressed high levels of p53 (data not shown). These data strongly suggest that pTpT increases the transcriptional activity of p53.

### EXAMPLE 8 Enhancement of DNA Repair

Expression of a UV-damaged reporter plasmid containing the bacterial chloramphenicolacetyltransferase (CAT) gene under the control of SV40 promoter and enhancer sequences, previously shown to detect decreased DNA repair capacity in human lymphocytes associated with aging and early-onset skin cancers (Wei, Q. et al., Proc. Natl. Acad. Sci.l USA 90:1614-1618 (1993)), was used to measure the DNA repair capacity of normal neonatal human skin-derived fibroblasts and keratinocytes.

Newborn keratinocytes were established as described (Stanulis-Praeger, B.M. and Gilchrest, B.A., J. Cell. Physiol. 139:116-124 (1989)) using a modification of the method of Rheinwald and Green (Gilchrest, B.A. et al., J. Invest. Dermatol. 101:666-672 (1993)). First-passage keratinocytes were maintained in a non-differentiating low Ca²⁺ medium (K-Stim, Collaborative Biomedical Products, Bedford, MA). Fibroblasts were established from dermal explants as described (Rheinwald, J.G. and Green, J., Cell 6:331-343 (1975)) and maintained in DMEM supplemented with 10% bovine serum. Cells were treated with either 100 µM pTpT or an equal volume of diluent (DMEM) for five days prior to transfection. Duplicate cultures of each condition were transfected using the Lipofectin Reagent Kit (GIBCO/BRL) and 5 µg reporter DNA, pCAT-control vector (Promega, Madison, WI). Before transfection, the vector DNA was either sham irradiated or exposed to 100 mJ/cm² UVB radiation from a 1 KW Xenon arc solar simulator (XMN 1000-21, Optical Radiation, Azuza, CA) metered at 285 ± 5 nm using a research radiometer (model IL 1700A, International Light, Newburyport, MA), as described (Yaar, M. et al., J. Invest. Dermatol. 85:70-74 (1985)). Cells were collected 24 hours after transfection in a lysis buffer provided in the CAT Enzyme Assay System (Promega, Madison, WI) using a protocol provided by the manufacturer. CAT enzyme activity was determined using the liquid scintillation counting protocol and components of the assay system kit. Labeled chloramphenicol [50-60 mC1 (1.85-2.22 GBq) mmol] was purchased from New England Nuclear (Boston, MA). Protein concentration in the cell extracts was determined by the method of Bradford (Anal. Biochem. 72:248 (1986)). CAT activity was expressed as c.p.m./100 µg protein and is represented as percent activity of cells transfected with sham-irradiated plasmid.

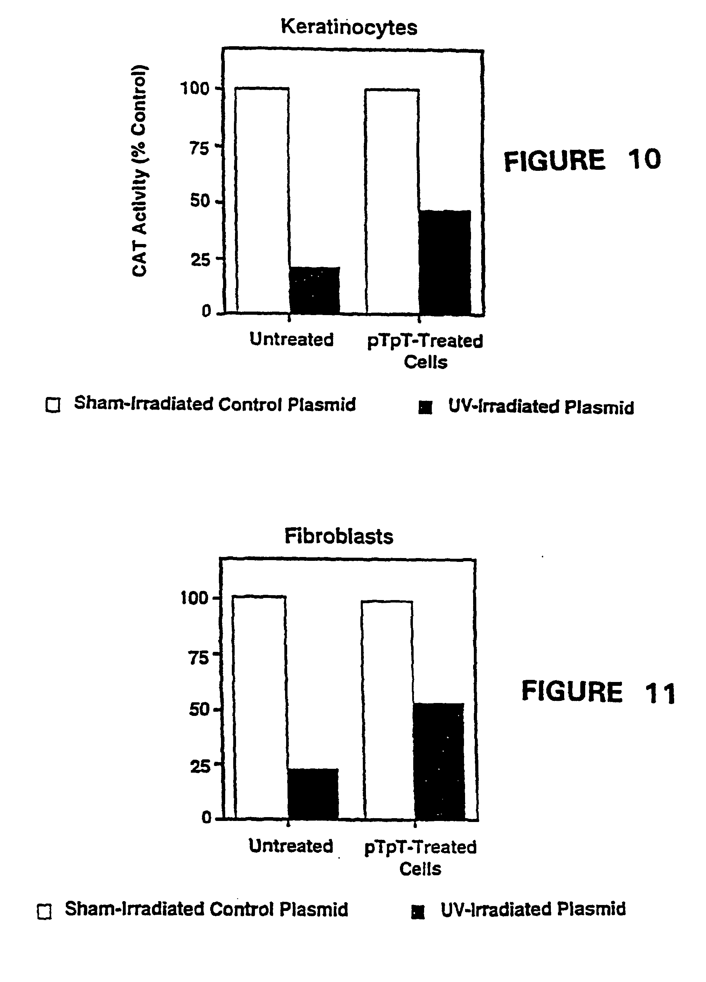

In preliminary experiments, exposure of the plasmid to a dose of solar-simulated irradiation (100 mJ/cm², metered at 285 nm) prior to transfection was identified as resulting in approximately 75% reduction in CAT activity assayed in cell lysates 16-24 hours after transfection, compared to that of sham-irradiated plasmid transfected into paired cultures. However, keratinocytes (Figure 10) and fibroblasts (Figure 11) pretreated with 100 µM pTpT for five days before transfection displayed CAT activity more than 50% that of sham-irradiated transfected controls. Because the reporter plasmid was nonreplicating, the level of CAT activity directly reflects the degree of DNA repair of the UV-damaged CAT gene restoring its biological activity. These data thus indicate that pTpT treatment of normal human fibroblasts and keratinocytes more than doubles the capacity of cells to repair UV-induced DNA damage over a 24 hour period. Cultured human cells have been shown to repair greater than 70% of UV-induced photoproducts within 24 hours after irradiation ((Mitchell, D.L. et al., Environmental UV Photobiology (Young, A.R. et al., eds), 345-377 (Plenum Press, New York and London, 1993)). The enhanced expression of Uv-irradiated plasmid in pTpT-treated cells did not result from a general increase in plasmid transcription in these cells, because the expression of the sham-irradiated plasmid was not higher than in non-pTpT-treated cells.

## Claims

1. Use of dinucleotides or dinucleotide dimers selected from the group consisting of: d(pT)₂, d(pC)₂, d(pA)₂, d(pCpT), d (pTpC), d (CpT), d(TpC) and d(TpT) for the manufacture of a medicament for the treatment in a mammal (e.g. a human) of hyperproliferative disease affecting epithelial cells, psoriasis, or atopic dermatitis.

2. The use according to claim 1, wherein the dinucleotides or dinucleotide dimmers are present in a delivery vehicle.

3. The use of Claim 2, wherein the delivery vehicle comprises (a) liposomes and/or (b) propylene glycol, optionally additionally comprising diacyl glycerol.

4. The use of any one of the preceding Claims, wherein the epithelial cells are carcinoma cells or skin cells, and the mammal is a human.

5. The use of any one of the preceding Claims, wherein the treatment is for hyperproliferative disease affecting epithelial cells and the dinucleotides or dinucleotide dimers are administered orally and/or by aerosol.

6. The use of any one of Claims 1-4, wherein the treatment comprises topical administration of the medicament.

## Patentansprüche

1. Verwendung von Dinukleotiden oder Dinukleotiddimeren, ausgewählt aus der Gruppe bestehend aus d(pT)₂, d(pC)₂, d(pA)₂, d (pCpT) , d(pTpC), d(CpT), d(TpC) und d (TpT) , für die Herstellung eines Medikaments zur Behandlung einer die Epithelzellen befallenden hyperproliferativen Erkrankung, Psoriasis oder atopische Dermatitis, bei einem Säugetier (z.B. einem Menschen).

2. Verwendung nach Anspruch 1, wobei die Dinukleotide oder Dinukleotiddimere in einem Zuführungsträger vorhanden sind.

3. Verwendung nach Anspruch 2, wobei der Zuführungsträger (a) Liposome und/oder (b) Propylenglykol und optional zusätzlich Diacylglycerol umfasst.

4. Verwendung nach einem der vorherigen Ansprüche, wobei die Epithelzellen Karzinomzellen oder Hautzellen sind und das Säugetier ein Mensch ist.

5. Verwendung nach einem der vorherigen Ansprüche, wobei die Behandlung für eine die Epithelzellen befallende hyperproliferative Erkrankung ist und die Dinukleotide oder Dinukleotiddimere oral und/oder per Aerosol verabreicht werden.

6. Verwendung nach einem der Ansprüche 1 bis 4, wobei die Behandlung die topische Verabreichung des Medikaments umfasst.

## Revendications

1. Utilisation de dinucléotides ou de dinucléotides dimères sélectionnés parmi le groupe constitué de : d(pT)₂, d(pC)₂, d(pA)₂, d(pCpT), d(pTpC), d(CpT), d(TpC) et d(TpT) pour la fabrication d'un médicament pour le traitement d'un mammifère (p.ex. un humain) contre une maladie hyperproliférative touchant les cellules épithéliales, de psoriasis, ou de dermatite atopique.

2. L'utilisation selon la revendication 1, les dinucléotides ou dinucléotides dimères étant présents dans un véhicule de délivrance.

3. L'utilisation de la revendication 2, le véhicule de délivrance comprenant (a) des liposomes et/ou (b) du propylène glycol, et comprenant optionnellement en plus du diacylglycérol.

4. L'utilisation de n'importe laquelle des revendications précédentes, les cellules épithéliales étant des cellules cancéreuses ou des cellules cutanées, et le mammifère étant humain.

5. L'utilisation de n'importe laquelle des revendications précédentes, le traitement étant contre une maladie hyperproliférative touchant les cellules épithéliales et les dinucléotides ou dinucléotides dimères étant administrés oralement et/ou par aérosol.

6. L'utilisation de n'importe laquelle des revendications 1 à 4, le traitement comprenant +l'administration locale du médicament.
